# EUROPEAN PATENT SPECIFICATION

(11) **EP 2 720 750 B1**
(45) Date of publication and mention of the grant of the patent: **04.10.2017**
(21) Application number: 12730120.8
(22) Date of filing: 19.06.2012
(51) Int. Cl.: A61N 1/36, H04R 25/00

(54) **VESTIBULAR IMPLANT SYSTEM WITH LOW BATTERY ALERT**
VORHOFIMPLANTATSYSTEM MIT ALARM FÜR GERINGEN BATTERIELADESTAND
SYSTÈME D'IMPLANT VESTIBULAIRE DOTÉ D'UNE ALERTE DE PILE FAIBLE

(30) Priority: 20.06.2011 US 201161498760 P
(43) Date of publication of application: 23.04.2014
(73) Proprietor: MED-EL Elektromedizinische Geräte GmbH, 6020 Innsbruck (AT)
(72) Inventor: JÄGER, Andreas, 6103 Reith bei Seefeld (AT); GARNHAM, Carolyn, Matlock Derbyshire DE4 3BZ (GB); HESSLER, Roland, 6020 Innsbruck (AT); ZIMMERLING, Martin, 6082 Patsch (AT); DELLA SANTINA, Charles Coleman, Towson, MD 21286 (US); FRIDMAN, Gene, Santa Clarita, CA 91350 (US)
(74) Representative: Lucke, Andreas
(86) International application number: PCT/US2012/043069
(87) International publication number: WO 2012/177589

(56) References cited:
- WO-A2-01/60282
- WO-A2-2004/045242
- WO-A2-2011/015673
- US-A1- 2002 072 781
- US-A1- 2007 027 676

## Description

### TECHNICAL FIELD

The present invention relates to implantable stimulation systems, and more specifically to a vestibular implant system with a low battery alert functionality.

### BACKGROUND ART

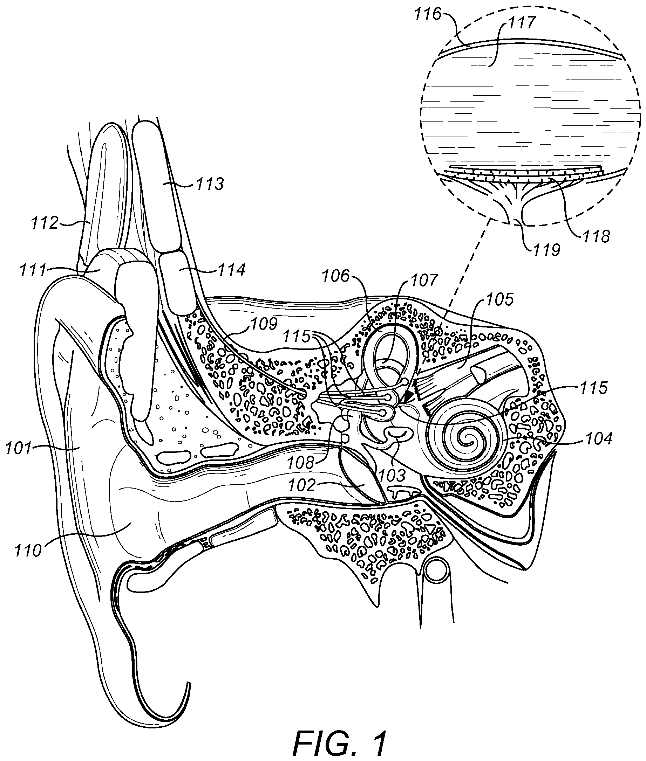

A normal ear directs sounds as shown in Figure 1 from the outer ear pinna **101** through the generally cylindrical ear canal **110** to vibrate the tympanic membrane **102** (eardrum). The tympanic membrane **102** moves the bones of the middle ear **103** (malleus, incus, and stapes) that vibrate the cochlea **104,** which in turn functions as a transducer to generate electric pulses to the brain that are interpreted as sounds.

In addition, the inner ear also includes a balance sensing vestibular system which involves the vestibular labyrinth, its three interconnected and mutually orthogonal semi-circular canals: the superior canal **106,** posterior canal **107,** and horizontal canal **108** (as well as the otolith organs **116** in the utricle and saccule of the inner ear. The canals and otoliths of the vestibular labyrinth contain hair cells **118** in a viscous endolymph **117** to sense head orientation and head movements, thereby activating vestibular nerve fibers **119** that send an electrical balance signal to the brain **105.**

In some people, the vestibular system is damaged or impaired. Such vestibular dysfunction can cause balance problems such as unsteadiness, vertigo and unsteady vision. This can be a significant handicap in everyday life. To treat such problems, Electrical stimulation of the vestibular system can help to restore the balancing function, and vestibular implants are currently under development to provide such an artificial balance signal.

Fig. 1 also shows some components of a vestibular implant system such as is described in U.S. Patent Application 61/366,345 (incorporated herein by reference). An external movement signal (from one or more sensors not shown) is processed by an external processor **111** to produce a vestibular stimulation signal. An external transmitter coil **112** couples the stimulation signal through the skin to an implanted receiver coil **113.** Implanted vestibular stimulator **114** than delivers the stimulation signal through an electrode lead **109** to vestibular stimulator electrodes **115** that electrically stimulate target neural tissue such as the semicircular canals **106, 107, 108,** one or both otolith organs, and/or the vestibular nerve **105** or ganglion for vestibular sensation by the patient as a balance signal.

One of the challenges for vestibular implant systems is the relatively significant amount of power required by the system. Gyro-based movement sensors have relatively high power consumption, and this requires a relatively large battery either in the implanted part or in an external part of the vestibular implant system and/or relatively frequent battery recharging cycles. Both the onset of electrical stimulation when the vestibular implant is being activated and the ending of electrical stimulation when the system switches off (e.g. when the battery is depleted) will be required from time to time. Both are challenging situations since the respective changes in electrical stimulation patterns can result in severe patient discomfort (e.g., vertigo). Certain situations, such as changes in the electrical stimulation patterns, can also be potentially dangerous; for example, when electrical stimulation of the vestibular organ suddenly stops while the patient is driving a car, especially when this occurs unexpectedly. Thus it is important for the patient to be alerted well in advance before the battery stops functioning and electrical stimulation of the vestibular system is switched off.

US 2002/0072781 discloses an apparatus and a method in which the portions of the labyrinth associated with the labyrinthine sense and the nerves associated therewith are stimulated to perform augmentation or control of a patient's respiratory function, opening a patient's airway, inducing sleep and counteracting vertigo. The system comprises a stimulation element that performs the actual stimulation of the tissue, a sensor detector for detecting a physiological condition of the patient and a power and control unit that receives the signals provided by the sensor and causes stimulation energy to be provided to the stimulation element at an appropriate timing, level, pattern and frequency to achieve the desired function. When the system is employed for augmenting and controlling a patient's respiratory function, alarms are provided and communicated to a caregiver so that an emergency condition, such as a failure of the vestibular stimulation system can be detected and reported.

WO 2001/015673 discloses a method of operating a hearing instrument, such as a cochlear implant. As long as a high battery status is detected, a standard audio signal processing mode is selected. Once a low battery status has been detected, a low power audio signal processing mode is selected, having reduced power consumption compared to the standard audio signal processing mode in order to extend the battery lifetime. In the low power audio signal processing mode, various audio signal processing functions can be switched off for at least part of the audio frequencies, such as feedback cancelling, auditory scene classification, frequency compression, noise reduction, pinna simulation and acoustic beam forming. In addition, part of the processor or a memory of a digital audio signal processing unit can be switched off on a regular temporary basis for reducing the duty cycle thereof to save energy.

WO 01/6028282 discloses a balance prosthesis system comprising a motion sensing system to be worn by a wearer, generating a motion signal indicative of the motion experienced by the body part of the wearer, a signal processor in communication with the motion sensing system for generating an estimate of the spatial orientation of the body part, an encoder in communication with the signal processor and configured to generate a feedback signal on the basis of the estimate of the spatial orientation, and a stimulator responsive to the feedback signal from the encoder and configured to provide a signal to the nervous system of the wearer in response to the feedback signal. This way, a wearer having a dysfunctional vestibular system may be provided with information indicative of the spatial orientation of the body part. The same document further suggests that the balance prosthesis functions as a sensory enhancer for extending the wearer's range of sensitivity. For this purpose, the encoder generates a zero feedback signal when the motion of the body part is such that it would be detectable by the wearer's vestibular system, and that is nonzero when the motion would otherwise be undetectable by the wearer's vestibular system.

### SUMMARY

Embodiments of the present diclosure are directed to a vestibular implant system and corresponding method of operating such a system. An implantable vestibular stimulator provides a vestibular stimulation signal to electrically stimulate target neural tissue for vestibular sensation by a patient. A patient warning alarm process alters the stimulation signal when a given alarm condition occurs to change the vestibular sensation of the patient thereby warning the patient of the alarm condition.

In further specific embodiments, the alarm condition may presage a change in operating functionality of the system whereby the altered stimulation signal warns the patient of the change. For example, the change in operating functionality may include a reduction in operating functionality of the system and/or a termination of operating functionality of the system.

The alarm condition may include a low battery condition, and/or a reduced or lost connection with an external portion of the system. There may be multiple different alarm conditions such that the alarm process alters the stimulation signal in a different distinctive way for each different alarm condition. The alarm process may alter the stimulation signal to a constant pacing mode instead of depending on sensory inputs. In addition or alternatively, the alarm process may alter the stimulation signal for a brief period to warn the patient of the alarm condition and then shift back to allow an unaltered stimulation signal. In such an arrangement, it may be useful to repeat the brief period of altering multiple times. And the alarm process may terminate the stimulation signal during the brief period of altering.

Embodiments of the present disclosure also are directed to another vestibular implant system and corresponding method of operating such a system. An implantable vestibular stimulator provides a vestibular stimulation signal to electrically stimulate target neural tissue for vestibular sensation by a patient. A patient warning alarm process activating a patient alarm signal on a separate sensory channel independent of the stimulation signal when a given alarm condition occurs to warn the patient of the alarm condition.

In further such embodiments, the alarm condition may presage a change in operating functionality of the system whereby the alarm signal warns the patient of the change. For example, the change in operating functionality may include a reduction in operating functionality of the system or a termination of operating functionality of the system. The alarm condition may include a low battery condition, or a reduced or lost connection with an external portion of the system. There may be multiple different alarm conditions with a different distinctive patient alarm signal for each alarm condition. The patient alarm signal may be routed via other sensory systems of the patient than the vestibular organ. The alarm signal can be a vibratory signal, an auditory signal, a visual signal or an electrical stimulation signal. Alarm signals can be elicited by an externally worn unit of the vestibular implant system and/or by its implantable unit.

The invention is set out in independent claim 1. Various embodiments of the invention are subject of the dependent claims.

### BRIEF DESCRIPTION OF THE DRAWINGS

Figure 1 shows various anatomical structures in a human ear including one specific embodiment of a vestibular implant system.

### DETAILED DESCRIPTION

Embodiments of the present invention are directed to a patient warning alarm for a vestibular implant system warning the patient of one or more alarm conditions such as low battery power. Various specific mechanisms are available, some of which are private to the patient and others of which are public and can intentionally also be recognized by persons in the vicinity of the patient.

For example, a private patient warning arrangement can be implemented in an implantable vestibular stimulator **114** that provides a vestibular stimulation signal to electrically stimulate target neural tissue of semi-circular canals **(106, 107, 108)** and/or otolith organs **116** for vestibular sensation by a patient. A patient warning alarm process (e.g., a software process running within the vestibular stimulator **114**) then alters the stimulation signal when a given alarm condition occurs to change the vestibular sensation of the patient thereby warning the patient of the alarm condition.

For example, the alarm condition may include a low battery condition, and/or a reduced or lost connection with an external portion of the system. Another alarm condition may presage a change in operating functionality of the system such as a reduction in operating functionality of the system and/or a termination of operating functionality of the system whereby the altered stimulation signal warns the patient of the change. Thus, in some embodiments there may be multiple different alarm conditions such that the alarm process alters the stimulation signal in a different distinctive way for each different alarm condition.

The alarm process may alter the stimulation signal to a constant pacing mode of the vestibular stimulator **114** independent of sensory inputs. In addition or alternatively, the alarm process may alter the stimulation signal from the vestibular stimulator **114** for a brief period to warn the patient of the alarm condition and then shift back to allow an unaltered stimulation signal. In such an arrangement, it may be useful to repeat the brief period of altering multiple times. And the alarm process may terminate the stimulation signal from the vestibular stimulator **114** during the brief period of altering.

A private patient warning arrangement can be implemented in an implantable vestibular stimulator **114** that provides a vestibular stimulation signal to electrically stimulate target neural tissue of semi-circular canals **(106, 107, 108)** or otolith organs **116** for vestibular sensation by a patient. A patient warning alarm process (e.g., a software process running within the vestibular stimulator **114**) then activates a patient alarm signal on a separate sensory channel independent of the stimulation signal when a given alarm condition occurs to warn the patient of the alarm condition. For example, the alarm condition may include a low battery condition, and/or a reduced or lost connection with an external portion of the system. Another alarm condition may presage a change in operating functionality of the system such as a reduction in operating functionality of the system and/or a termination of operating functionality of the system whereby the altered stimulation signal warns the patient of the change. Thus, in some embodiments there may be multiple different alarm conditions such that the alarm process alters the stimulation signal in a different distinctive way for each different alarm condition. The patient alarm signal may be a vibratory signal, an auditory signal, a visual signal or an electrical stimulation signal.

In the foregoing, references to vestibular implant systems should be understood broadly to include all implantable arrangements that provide stimulation signals affecting the balance sensing system. Specifically such arrangements may or may not include motion sensors, whether internal or external. For example, a vestibular implant system without motion sensing signals may be useful for treatment related to Meniere's disease and may be thought of as a Meniere's implant. And vestibular implant arrangements may also be integrated together with other related implantable systems such as middle ear implants, cochlear implants, bone conduction implants, auditory brainstem implants, etc. And the stimulation signals may occur either by electrical means as commonly used in current cochlear implant technology, by optical means (e.g. as disclosed in US patent 7,488,341 and in US patent application 12/368,548; by mechanical means (e.g. as disclosed in US patent application 11/193,034, and or some combination of different types of stimulation signals. And specific alarm signals and alarm conditions in any of the described variations can be combined.

Embodiments of the disclosure may be implemented in part in any conventional computer programming language. For example, preferred embodiments may be implemented in a procedural programming language (*e.g.*, "C") or an object oriented programming language (*e.g.*, "C++", Python). Alternative embodiments of the invention may be implemented as pre-programmed hardware elements, other related components, or as a combination of hardware and software components.

Embodiments can be implemented in part as a computer program product for use with a computer system. Such implementation may include a series of computer instructions fixed either on a tangible medium, such as a computer readable medium (*e.g*., a diskette, CD-ROM, ROM, or fixed disk) or transmittable to a computer system, via a modem or other interface device, such as a communications adapter connected to a network over a medium. The medium may be either a tangible medium (*e.g*., optical or analog communications lines) or a medium implemented with wireless techniques (*e.g*., microwave, infrared or other transmission techniques). The series of computer instructions embodies all or part of the functionality previously described herein with respect to the system. Those skilled in the art should appreciate that such computer instructions can be written in a number of programming languages for use with many computer architectures or operating systems. Furthermore, such instructions may be stored in any memory device, such as semiconductor, magnetic, optical or other memory devices, and may be transmitted using any communications technology, such as optical, infrared, microwave, or other transmission technologies. It is expected that such a computer program product may be distributed as a removable medium with accompanying printed or electronic documentation (*e.g*., shrink wrapped software), preloaded with a computer system (*e.g*., on system ROM or fixed disk), or distributed from a server or electronic bulletin board over the network (*e.g*., the Internet or World Wide Web).

The invention is defined in the appended claims.

## Claims

1. A vestibular implant system comprising:
an implantable vestibular stimulator (114) configured to provide a vestibular stimulation signal
to electrically stimulate target neural tissue (106, 107, 108) for vestibular sensation by a patient; **characterized by**
a patient warning alarm process configured to activate a patient alarm signal on a separate sensory channel independent of the stimulation signal when a given alarm condition occurs,
thereby warning the patient of the alarm condition.

2. A system according to claim 1, wherein the alarm condition presages a change in operating functionality of the system whereby the altered stimulation signal warns the patient of the change.

3. A system according to claim 2, wherein the change in operating functionality includes a reduction in operating functionality of the system.

4. A system according to claim 2, wherein the change in operating functionality includes a termination of operating functionality of the system.

5. A system according to claim 1, wherein the alarm condition includes a low battery condition.

6. A system according to claim 1, wherein the alarm condition includes a reduced connection with an external portion of the system.

7. A system according to claim 1, wherein the alarm condition includes loss of connection with an external portion of the system.

8. A system according to claim 1, wherein the given alarm condition includes a plurality of different alarm conditions, and wherein the alarm process alters the stimulation signal in a different distinctive way for each alarm condition.

9. A system according to claim 1, wherein the alarm process alters the stimulation signal to a constant pacing mode independent of sensory inputs.

10. A system according to claim 1, wherein the alarm process alters the stimulation signal for a brief period to warn the patient of the alarm condition and then shifts back to allow an unaltered stimulation signal.

11. A system according to claim 10, wherein the alarm process repeats the brief period of altering a plurality of times.

12. A system according to claim 10, wherein the alarm process terminates the stimulation signal during the brief period of altering.

13. A system according to claim 1, wherein the patient alarm signal is a vibratory signal or an auditory signal.

14. A system according to claim 1, wherein the patient alarm signal is a visual signal.

15. A system according to claim 1, wherein the patient alarm signal is an electrical stimulation signal.

## Patentansprüche

1. Ein Vestibulaimplantatsystem, das folgendes umfasst:
einen implantierbaren Vestibulastimulator (114), der dazu konfiguriert ist, ein Vestibula-Stimulationssignal bereitzustellen, um Ziel-Nervengewebe (106, 107, 108) elektrisch zu stimulieren, damit ein Patient eine Vestibula-Empfindung erhält, **gekennzeichnet durch** einen Patientenwarnalarmprozess, der dazu konfiguriert ist, ein Patienten-Alarmsignal auf einem getrennten Sensorik-Kanal zu aktivieren, der unabhängig von dem Stimulationssignal ist, wenn ein gegebener Alarmzustand auftritt, um dadurch den Patienten vor dem Alarmzustand zu warnen.

2. System nach Anspruch 1, bei dem der Alarmzustand eine Änderungen in der Betriebsfunktionalität des Systems vorsieht, wobei das geänderte Stimulationssignal den Patienten vor der Änderung warnt.

3. System nach Anspruch 2, bei dem die Änderung in der Betriebsfunktionalität eine Verringerung in der Betriebsfunktionalität des Systems umfasst.

4. System nach Anspruch 2, bei dem die Änderung in der Betriebsfunktionalität eine Beendigung der Betriebsfunktionalität des Systems umfasst.

5. System nach Anspruch 1, bei dem der Alarmzustand einen Zustand niedriger Batterieleistung umfasst.

6. System nach Anspruch 1, bei dem der Alarmzustand eine eingeschränkte Verbindung mit einem externen Teil des Systems umfasst.

7. System nach Anspruch 1, bei dem der Alarmzustand den Verlust einer Verbindung mit einem externen Teil des Systems umfasst.

8. System nach Anspruch 1, bei dem der gegebene Alarmzustand eine Mehrzahl von Alarmzuständen umfasst, und wobei der Alarmprozess für jeden Alarmzustand das Stimulationssignal auf eine andere, unterschiedliche Weise ändert.

9. System nach Anspruch 1, bei dem der Alarmprozess das Stimulationssignal unabhängig von sensorischen Eingaben in einen konstanten Schritt-Modus wechselt.

10. System nach Anspruch 1, bei dem der Alarmprozess das Stimulationssignal für eine kurze Zeitspanne ändert, um den Patienten vor dem Alarmzustand zu warnen und dann zurückschaltet, um ein nicht verändertes Stimulationssignal zu gestatten.

11. System nach Anspruch 10, bei dem der Alarmprozess die kurze Zeitspanne, in der die Änderung geschieht, mehrmals wiederholt wird.

12. System nach Anspruch 10, bei dem der Alarmprozess das Stimulationssignal während der kurzen Zeitspanne, in der die Änderung stattfindet, stoppt.

13. System nach Anspruch 1, bei dem das Patientenalarmsignal ein Vibrationssignal oder ein hörbares Signal ist.

14. System nach Anspruch 1, bei dem das Alarmsignal ein visuelles Signal ist.

15. System nach Anspruch 1, bei dem das Patientenalarmsignal ein elektrisches Stimulationssignal ist.

## Revendications

1. Système d'implant vestibulaire comprenant :
un stimulateur vestibulaire implantable (114) configuré pour produire un signal de stimulation vestibulaire afin de stimuler électriquement un tissu neuronal cible (106, 107, 108) pour une sensation vestibulaire par un patient ; **caractérisé par**
un processus d'alarme d'avertissement du patient configuré pour activer un signal d'alarme du patient sur un canal sensoriel séparé indépendant du signal de stimulation quand une condition d'alarme donnée se produit, ce qui avertit le patient de la condition d'alarme.

2. Système selon la revendication 1, dans lequel la condition d'alarme annonce un changement du fonctionnement des fonctionnalités du système, le signal de stimulation modifié avertissant le patient de ce changement.

3. Système selon la revendication 2, dans lequel le changement du fonctionnement des fonctionnalités comprend une réduction du fonctionnement des fonctionnalités du système.

4. Système selon la revendication 2, dans lequel le changement du fonctionnement des fonctionnalités comprend une interruption du fonctionnement des fonctionnalités du système.

5. Système selon la revendication 1, dans lequel la condition d'alarme comprend une condition de faible niveau de pile.

6. Système selon la revendication 1, dans lequel la condition d'alarme comprend une réduction de la connexion avec une partie externe du système.

7. Système selon la revendication 1, dans lequel la condition d'alarme comprend une perte de la connexion avec une partie externe du système.

8. Système selon la revendication 1, dans lequel la condition d'alarme donnée comprend une pluralité de conditions d'alarme différentes et dans lequel le processus d'alarme modifie le signal de stimulation d'une façon distinctive différente pour chaque condition d'alarme.

9. Système selon la revendication 1, dans lequel le processus d'alarme modifie le signal de stimulation selon un mode de cadencement constant indépendant des entrées sensorielles.

10. Système selon la revendication 1, dans lequel le processus d'alarme modifie le signal de stimulation pendant une brève période pour avertir le patient de la condition d'alarme, puis le rétablit pour permettre un signal de stimulation non modifié.

11. Système selon la revendication 10, dans lequel le processus d'alarme répète la brève période de modification une pluralité de fois.

12. Système selon la revendication 10, dans lequel le processus d'alarme interrompt le signal de stimulation pendant la brève période de modification.

13. Système selon la revendication 1, dans lequel le signal d'alarme du patient est un signal vibratoire ou un signal sonore.

14. Système selon la revendication 1, dans lequel le signal d'alarme du patient est un signal visuel.

15. Système selon la revendication 1, dans lequel le signal d'alarme du patient est un signal de stimulation électrique.
